# EUROPÄISCHE PATENTSCHRIFT

(11) **EP 1 115 681 B1**
(45) Veröffentlichungstag und Bekanntmachung des Hinweises auf die Patenterteilung: **19.03.2003**
(21) Anmeldenummer: 99952457.2
(22) Anmeldetag: 21.09.1999
(51) Int. Cl.: C07C 29/76, C07C 29/80

(54) **VERFAHREN ZUR HERSTELLUNG VON HOCHREINEM MONOETHYLENGLYKOL**
METHOD FOR PRODUCING HIGHLY PURE MONOETHYLENE GLYCOL
PROCEDE DE PRODUCTION DE MONOETHYLENE GLYCOL A HAUTE PURETE

(30) Priorität: 23.09.1998 DE 19843652
(43) Veröffentlichungstag der Anmeldung: 18.07.2001
(73) Patentinhaber: BASF AKTIENGESELLSCHAFT, 67056 Ludwigshafen (DE)
(72) Erfinder: ADRIAN, Till, D-67240 Bobenheim-Roxheim (DE); BESSLING, Bernd, D-67269 Grünstadt (DE); HASSE, Hans, D-67661 Kaiserslautern (DE); VANSANT, Frans, B-02920 Kalmthout (BE); THEIS, Gerhard, D-67133 Maxdorf (DE)
(74) Vertreter: Isenbruck, Günter, Dr.
(86) Internationale Anmeldenummer: EP9906967
(87) Internationale Veröffentlichungsnummer: WO00017140

(56) Entgegenhaltungen:
- EP-A- 0 032 665
- US-A- 4 622 104

## Beschreibung

Die Erfindung betrifft ein Verfahren zur Herstellung von hochreinem Monoethylenglykol.

Monoethylenglykol wird großtechnisch durch Hydrolyse von Ethylenoxid, Entwässerung und Reindestillation hergestellt. Zur Verbesserung der Selektivität der Ethylenoxid-(im folgenden abgekürzt als EO bezeichnet)-Hydrolyse wird der Hydrolysereaktor mit einem großen Wasserüberschuß (Gewichtsverhältnis Wasser:EO=4:1 bis 15:1) betrieben. Dadurch kann der Anteil an höheren Glykolen, insbesondere Diethylenglykol, Triethylenglykol usw. zurückgedrängt werden. Der Hydrolysereaktor wird üblicherweise bei Temperaturen von 120°C bis 250°C und Drücken von 30 - 40 bar betrieben. Das Hydrolyseprodukt wird zunächst entwässert, auf einen Restwassergehalt von 100 - 200 ppm und anschließend in die verschiedenen Glykole in reiner Form aufgetrennt.

Die Entwässerung erfolgt in der Regel in einer Kaskade von druckgestaffelten Kolonnen, mit abnehmendem Druck. Aus Gründen der Wärmeintegration wird in der Regel nur der Sumpfverdampfer der ersten Druckkolonne mit Fremddampf beheizt, alle weiteren Druckkolonnen dagegen mit den Brüden der jeweils voranstehenden Kolonne. Der Zulauf wird jeweils im Sumpf der Kolonnenen aufgegeben, unterhalb des ersten Bodens, da zur Trennung von Wasser und Glykolen kein Abtriebsteil notwendig ist. Je nach Wassergehalt des Hydrolysereaktoraustrags und des Druck/Temperaturniveaus des im Sumpfverdampfer der ersten Kolonne eingesetzten Fremddampfs besteht die Druckentwässerungskaskade aus 2 bis 7 Kolonnen. An die Druckentwässerung schließt sich eine Vakuumentwässerung an, die in der Regel in einer Kolonne mit Abtriebsteil erfolgt. Das bei der Entwässerung anfallende Wasser wird vor den Hydrolysereaktor zurückgeführt. Die entwässerte, Glykole enthaltende Lösung wird in mehreren Kolonnen in die Reinstoffe zerlegt. Die Produkte Mono-, Di- und Triethylenglykol werden jeweils als Kopfprodukt abgezogen, alle weiteren höheren Glykole fällen in Form eines Gemischs unter der Bezeichnung Polyethylenglykole als Sumpfprodukt der letzten Kolonne an.

Herkömmliche Glykolanlagen besitzen neben den Produktströmen üblicherweise nur einen einzigen weiteren Auslaß, den sogenannten Acetaldehydpurge am Sumpfverdampfer der zweiten Druckentwässerungskolonne. Dort wird der unkondensierte Anteil der zur Beheizung eingesetzten Brüden der ersten Kolonne ausgeschleust. Somit können Nebenkomponenten, die entweder mit dem Wasser/EO-Strom in die Glykolanlage eingetragen werden oder infolge von Nebenreaktionen in der Glykolanlage gebildet werden nur über den Acetaldehydpurge oder über die Produktströme auf der Anlage ausgeschleust werden. Letzteres beeinträchtigt die Produktqualität und ist somit unerwünscht.

Bislang wurden Glykolanlagen nur bezüglich ihrer Hauptaufgaben optimiert, insbesondere der Energie- und Investitionskostenreduzierung bei der Entwässerung und Reindestillation. In jüngster Zeit werden zunehmend höhere Anforderungen an die Produktqualität von Monoethylenglykol gestellt, insbesondere bezüglich des Gehalts an Nebenkomponenten. Es gibt zwei Monoethylenglykol-Produktqualitäten: Technical Grade (Antifreeze Grade) mit geringeren Anforderungen, für den Einsatz als Kühlmittel und Fiber Grade, mit strengen Anforderungen zum Einsatz in der Kunstfaserproduktion. Die Spezifikationen für Fiber Grade betragen, kundenabhängig, für freie Aldehyde, berechnet als Acetaldehyd, nach der spektrofotometrischen Bestimmungsmethode als blauer MBTH-Komplex 7 bis 20 ppm und Mindest-UV-Transmission bei 220 nm 76% - 80% und bei 275 nm 90% - 95%. Zum Meßwert für die freien Aldehyde tragen insbesondere Formaldehyd, Acetaldehyd und Glykolaldehyd bei. Die UV-aktiven Substanzen, sogenannte UV-Spoiler, sind weitgehend unbekannt und schon in Konzentrationen von weniger als 1 ppm spezifikationsschädlich. Beispiele sind Acrolein und Crotonaldehyd.

Aus Ullmanns Encyclopädie der technischen Chemie, 4. Auflage, Band 8, Seiten 200 bis 202 ist ein Verfahren zur großtechnischen Herstellung von Glykol mit den Verfahrensstufen der Reaktion des Ethylenoxids mit Wasser, Entwässerung des Glykol-Gemisches und Reindarstellung der einzelnen Glykole durch Rektifikation beschrieben. Die wässrige Glykollösung wird beispielsweise in einer mehrstufigen Verdampferanlage auf einen Restwassergehalt von ca. 10% aufkonzentriert und in einer weiteren, nachgeschalteten Vakuumkolonne vollständig entwässert. Da das dabei abgetrennte Wasser mit Aldehyden angereichert ist, ist ein gelegentlicher Austausch desselben erforderlich.

Die JP-A-60,089,439 beschreibt ein Verfahren zur Reinigung von Monoethylenglykol durch Vakuumdestillation unter Zufuhr von Inertgas. Mit dem Stickstoffstrom wird der überwiegende Teil der Nebenkomponenten ausgestrippt und ein hochreines Glykol, das für die Faserherstellung geeignet ist, erhalten. Das Verfahren hat jedoch den Nachteil, daß zur effektiven Ausschleusung von Nebenkomponenten große Mengen an Stickstoff benötigt werden. Dies führt zu unerwünschten Produktverlusten im Abgas und zu einer unzulässig starken fluiddynamischen Belastung der Destillationskolonne.

Die DE-A-1 942 094 beschreibt ein Verfahren zur Reinigung von Monoethylenglykolen durch Wasserdampfdestillation in einer Abtriebskolonne, wobei durch den Wasserdampf die Flüchtigkeit der Verunreinigungen in Bezug auf das Monoethylenglykol erhöht wird.

Die CA-C-133050 beschreibt ein Verfahren zur Reinigung von Monoethylenglykol durch Zusatz von Bisulfitionen und anschließender Behandlung mit Anionenaustauscherharzen.

Weiterhin sind Reinigungsverfahren für Monoethylenglykol bekannt, wobei die Bildung von Nebenkomponenten durch besondere Maßnahmen im Bereich der Apparatekonstruktion und der dabei verwendeten Werkstoffe reduziert werden soll. Die DE-A-19602116 beschreibt ein Reinigungsverfahren für Monoethylenglykol in einer Apparatur, deren Oberfläche mit reduzierenden Phosphorverbindungen behandelt ist. Die oben erwähnten Verfahren haben jedoch den Nachteil, daß Zusatzstoffe oder zusätzliche apparative Maßnahmen für die Gewinnung von hochreinem Monoethylenglykol notwendig sind.

Es ist Aufgabe der Erfindung, ein einfaches destillatives Verfahren zur Gewinnung von hochreinem Monoethylenglykol zur Verfügung zu stellen, ohne die Verwendung von Zusatzstoffen oder von speziellen Werkstoffen. Die Ausschleusung der spezifikationsschädlichen Nebenkomponenten soll in überwiegend wäßrigen Abfallströmen, mit Glykolgehalten von maximal 1 Gew.-% erfolgen, wobei die Nebenkomponenten in den Abfallströmen um den Faktor 10- 100 aufkonzentriert werden sollen, da ansonsten zuviel Abwasser produziert wird.

Die Lösung geht aus von einem Verfahren zur destillativen Gewinnung von hochreinem Monoethylenglykol aus dem Hydrolyseprodukt von Ethylenoxid durch Druckentwässerung, bevorzugt in einer Kaskade, Vakuumentwässerung und anschließender Reindestillation. Die Erfindung ist dann dadurch gekennzeichnet, daß die Druckentwässerung in einer Entwässerungskolonne mit Abtriebsteil mit mindestens einer Trennstufe, besonders bevorzugt 2-10 Trennstufen, besonders bevorzugt mit 3-6 Stufen, stattfindet und daß ein Teil des Kopfstroms der Entwässemngskolonne(n) mit Abtriebsteil ausgeschleust wird.

Es wurde gefunden, daß die spezifikationsschädlichen Nebenkomponenten an bestimmten Stellen im Verfahren besonders effektiv ausgeschleust werden können. Das Erkennen dieser Stellen im Verfahren ist nicht trivial, da das Verhalten der Nebenkomponenten aufgrund der komplexen Phasengleichgewichte bislang nicht ausreichend gut beurteilt werden konnte. Daher wurde in herkömmlichen großtechnischen Verfahren nur ein sehr grober Auslaß für extrem leichtsiedende Nebenkomponenten geschaffen, der sogenannte Acetaldehydpurge am Sumpfverdampfer der zweiten Druckentwässerungskolonne. Dieser Auslaß ist nicht optimiert, da das Verhalten der Nebenkomponenten weitgehend unbekannt war bzw. bei der Verfahrensgestaltung nicht berücksichtigt wurde.

Die Komponenten werden bezüglich ihrer Siedelage vorliegend in drei Klassen eingeteilt:
1. Leichtsieder, mit einer Flüchtigkeit unterhalb der von Wasser (insbesondere Acetaldehyd, Formaldehyd in reinem Wasser, Acrolein)
2. Mittelsieder mit einer Flüchtigkeit zwischen der von Wasser und Monoethylenglykol (insbesondere Formaldehyd in glykolhaltigen wässrigen Lösungen, Formaldehyd in wasserfreien Monoethylenglykol, Glykolaldehyd, Crotonaldehyd)
3. Schwersieder mit einer geringeren Flüchtigkeit als Monoethylenglykol (insbesondere höhermolekulane Aldehyde, UV-Spoiler).

Erfindungsgemäß wird die Ausschleusung von Nebenkomponenten, insbesondere von Leichtsiedern, in der Druckentwässerungsstufe verbessert. Dazu wird die Druckentwässerungskolonne oder mindestens die erste Druckentwässerungskolonne der Kaskade mit einem Abtriebsteil mit mindestens einer Trennstufe, bevorzugt 2 bis 10 Trennstufen, besonders bevorzugt mit 3 bis 6 Stufen ausgestattet, wobei ein Teil des Kopfstroms der Druckentwässerungskolonne(n) mit Abtriebsteil ausgeschleust wird.

Herkömmliche großtechnische Verfahren haben den sogenannten Acetaldehydpurge am Sumpfverdampfer der zweiten Druckentwässerungskolonne: hier werden die Brüden der ersten Druckentwässerungskolonne weitgehend kondensiert, der nicht kondensierte Anteil, etwa 1-5 Gew.-% der gesamten Brüden, wird ausgeschleust. Die Restbrüden werden ggf. in einem weiteren Wärmeübertrager nachkondensiert, wobei die Kondensationswärme an einer geeigneten Stelle im Gesamtprozeß genutzt werden kann. Nach dieser herkömmlichen Lösung können jedoch nur Nebenkomponenten, die in der ersten Druckentwässerungskolonne als Bestandteil der Brüden abgeführt werden, über den Acetaldehydpurge ausgeschleust werden. Dies ist insbesondere im Fall von Formaldehyd unzureichend, da die Flüchtigkeit von Formaldehyd in wässrigen Glykollösungen mit Zunahme des Glykolgehalts abnimmt, insbesondere infolge von chemischen Reaktionen des Formaldehyds mit Wasser und Glykolen. Um somit Formaldehyd aus dem glykolhaltigen Sumpfprodukt der Druckentwässerungskolonne abzutrennen, ist ein Abtriebsteil in der Druckentwässerungskolonne bzw. mindestens in der ersten Druckentwässerungskolonne einer Kaskade mit mindestens einer Trennstufe, bevorzugt 2 bis 10 Trennstufen, besonders bevorzugt 3 bis 6 Trennstufen notwendig. Erst wenn der Formaldehyd in die rein wässrigen Brüden der ersten Kolonne abgetrennt worden ist, kann er zusammen mit Acetaldehyd über den Acetaldehydpurge ausgeschleust werden. Dabei gelingt die Abtrennung des Formaldehyds im Abtriebsteil umso besser, je höher die Temperatur und entsprechend der Druck in der Druckentwässerungskolonne bzw. in der ersten Druckentwässerungskolonne der Kaskade ist und je mehr Wasser im Reaktoraustrag vorhanden ist. Zwei der zusätzlichen Böden im Abtriebsteil können eingespart werden, wenn der Sumpfverdampfer konstruktiv mit "getrenntem Sumpf' entsprechend der DE-C-3338488 ausgeführt ist.

Die Menge der ausgeschleusten Nebenkomponenten, inbesondere Acetaldehyd und Formaldehyd, hängt davon ab, wieviel Abwasser ausgeschleust wird. Dabei kann aber die Menge des im Sumpfverdampfer der zweiten Entwässerungskolonne nicht kondensierten Brüdens aus Gründen des Energieverbundes und aus regelungstechnischen Randbedingungen nicht beliebig gesteigert werden. Es wurde eine besonders bevorzugte Verfahrensvariante gefunden, wonach eine weitere Abtrennung von Nebenkomponenten aus dem kondensierten Brüden durch Dampfstrippung möglich ist. Der mit Nebenkomponenten beladene Strippdampf kann anschließend an einer geeigneten Stelle im Prozeß energetisch genutzt werden. Zur Wasserdampfstrippung wird daher keine zusätzliche Energie benötigt, sondern lediglich ein zusätzlicher Apparat. Die Ausschleusung von Nebenkomponenten ist besonders wirkungsvoll, wenn der Ablauf des Strippers als Rücklauf auf die erste Entwässerungskolonne gegeben wird, da durch diese Rückführung der Aldehydgehalt am Kopf der ersten Druckentwässerungskolonne und im Stripper steigt und damit auch die Ausschleusrate zunimmt.

In vorteilhafter Weise beträgt die Temperatur unterhalb der Zulaufstelle über 80°C, bevorzugt zwischen 100°C und 250°C, besonders bevorzugt zwischen 115°C und 230°C. Der Druck im Abtriebsteil liegt bei mindestens 1 bar, bevorzugt bei 2-30 bar.

Vorteilhaft wird der Kopfstrom der Entwässerungskolonne(n) mit Abtriebsteil in einen Teilkondensator und/oder einen Stripper, insbesondere einen Wasserdampfstripper, eingeleitet und der (die) an der Nebenkomponenten angereichterte(n) gasförmige Strom (Ströme) ausgeschleust.

In geeigneter Weise werden der Teilkondensator und/oder der Stripper bei Temperaturen über 90°C, bevorzugt zwischen 120°C und 250°C, betrieben.

Die Erfindung wird im folgenden anhand einer Zeichnung sowie von Ausführungsbeispielen näher erläutert.

Es zeigen im einzelnen:
- **Figur 1**: ein Schema für ein großtechnisches Verfahren zur Glykolgewinnung nach dem Stand der Technik,
- **Figur 2**: ein Schema eines besonders bevorzugten Verfahrens zur Glykolgewinnung gemäß der Erfindung,
- **Figur 3**: ein Ausführungsbeispiel für ein erfindungsgemäßes Verfahren mit einer Druckentwässerungskolonne mit Abtriebsteil und einem Auslaß für Nebenkomponenten als Kopfstrom sowie anschließender Konzentrierung in einem Teilkondensator und einem Stripper.

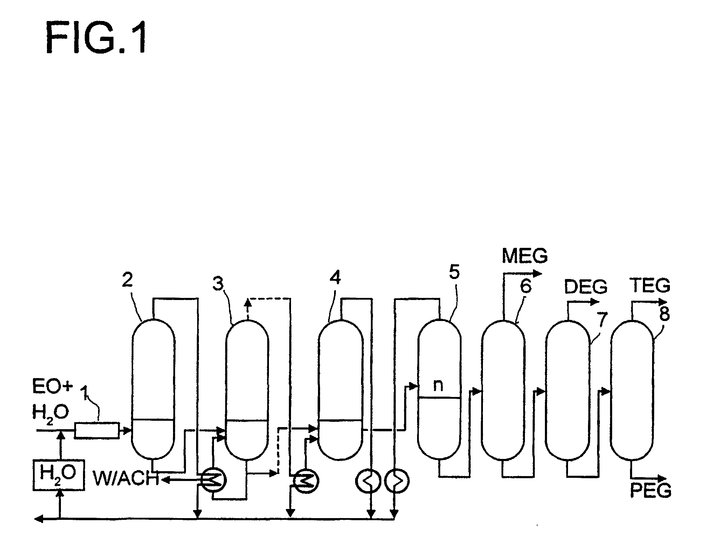

Figur 1 zeigt ein Schema für die großtechnische Gewinnung von Glykol nach dem Stand der Technik. Ein Wasser/Ethylenoxid-Gemisch mit einem Gewichtsverhältnis Wasser:Ethylenoxid von 4:1 bis 15:1 wird dem Hydrolysereaktor 1 zugeführt und anschließend einer Druckentwässerung, die vorliegend als Kaskade mit drei druckgestaffelten Kolonnen 2, 3 und 4 dargestellt ist. Der Zulauf der Kolonnen 2, 3 und 4 liegt jeweils im Sumpf. Der Brüdenstrom der ersten Druckentwässerungskolonne 2 wird im Sumpfverdampfer der zweiten Druckentwässerungskolonne 3 kondensiert und der nicht kondensierte Anteil als sogenannter Acetaldehydpurge (W/ACH, d.h. Wasser/Acetaldehyd) ausgeschleust. Die kondensierten Brüden der Druckentwässerungskolonnen 2, 3 und 4 werden vor den Hydrolysereaktor 1 zurückgeführt. Der Sumpfstrom der letzten Druckentwässerungskolonne 4 wird einer Vakuumentwässerungskolonne 5 in deren mittleren Teil aufgegeben. Der überwiegend wasserhaltige Brüden aus der Vakuumentwässerungskolonne 5 wird ebenfalls kondensiert und vor den Hydrolysereaktor 1 zurückgeführt. Der Sumpfaustrag der Vakuumentwässerungskolonne 5 wird einer Monoethylenglykolreindestillationskolonne 6 zugeführt, aus der als Kopfprodukt Monoethylenglykol, sowie Nebenkomponenten, insbesondere Formaldehyd, Glykolaldehyd und UV-Spoiler, abgezogen werden. Der Sumpfaustrag der Monoethylenglykolreindestillationskolonne 6 wird einer Diethylenglykol-Reindestillationskolonne 7 zugeführt, aus der als Kopfprodukt reines Diethylenglykol abgezogen und deren Sumpfaustrag einer weiteren Kolonne, der Triethylenglykol-Reindestillationskolonne 8 zugeführt wird. Das Kopfprodukt der Triethylenglykol-Reindestillationskolonne ist reines Triethylenglykol und der Sumpfaustrag der Kolonne 8 enthält eine Mischung höherer Glykole, die als "Polyethylenglykol" bezeichnet wird.

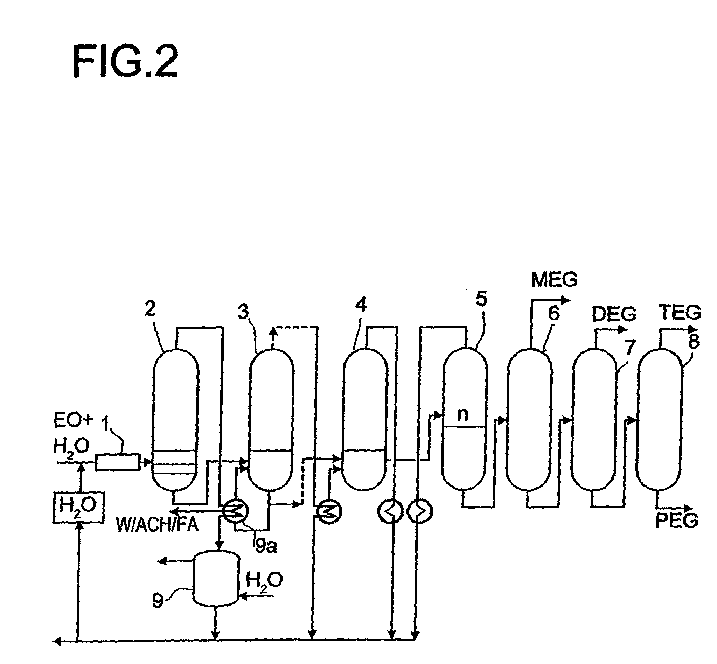

Figur 2 zeigt demgegenüber ein großtechnisches Verfahren zur Gewinnung von hochreinem Monoethylenglykol nach der Erfindung: Gegenüber dem Verfahrensschema in Figur 1 ist der Zulauf zur ersten Druckentwässerungskolonne 2 höhergelegt, und zwar weist diese Druckentwässerungskolonne 2 einen Abtriebsteil mit 2 bis 6 Böden auf.

Ein weiterer Unterschied zum Verfahren nach Figur 1 besteht darin, daß der Brüden der ersten Druckentwässerungskolonne 2 nach Teilkondensation im Sumpfverdampfer der Druckentwässerungskolonne 3 in einem Stripper 9 mit Wasserdampf von Nebenkomponenten freigestrippt wird. Aus dem Stripper wird ein Nebenkomponenten enthaltender gasförmiger Strom (W/ACH/FA, d.h. Wasser/Acetaldehyd/Formaldehyd) ausgeschleust.

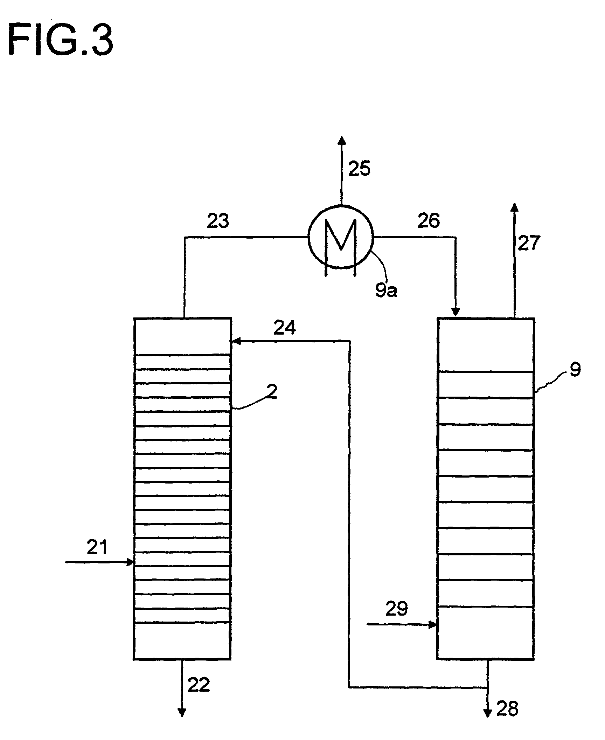

Figur 3 zeigt ein Beispiel für die erfindungsgemäße Ausgestaltung einer Druckentwässerungskolonne 2 mit Abtriebsteil sowie mit einem Stripper 9 zur Aufkonzentrierung der Nebenkomponenten vor deren Ausschleusung. Der Zulauf 21 des aufzutrennenden glykolhaltigen Stroms liegt auf dem 5. Boden einer Druckentwässerungskolonne 2 mit 20 Glockenböden, deren Kopfstrom 23 wird nach Teilkondensation als Strom 26 auf einen Stripper 9 mit 10 Glockenböden gegeben, und im Gegenstrom mit Wasserdampf 29 von Nebenkomponenten freigestrippt. Die Nebenkomponenten enthaltenden gasförmigen Ströme 25 und 27 werden ausgeschleust. Ein Teilstrom des Sumpfaustrags des Strippers 9 bildet als Teilstrom 24 den Rücklauf der Entwässerungskolonne 2. Die Zusammensetzung der Ströme 21-29 ist für ein Verfahren nach der Erfindung in Tabelle 1a aufgeführt. Zum Vergleich ist die Zusammensetzung der Ströme 21-29 für ein Verfahren nach dem Stand der Technik, d.h. mit Druckentwässerungskolonne ohne Abtriebsteil und ohne Stripper in Tabelle 1b aufgeführt.

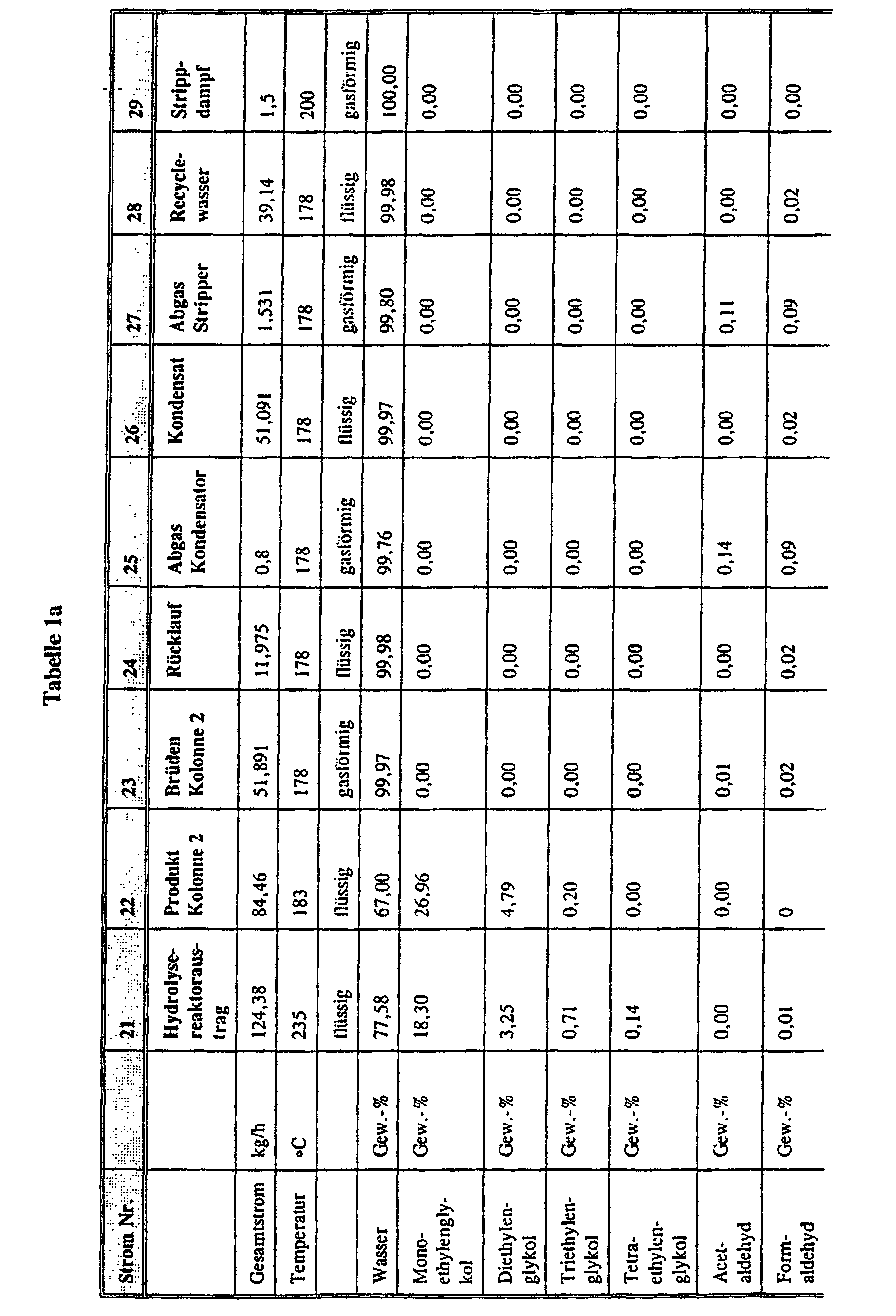

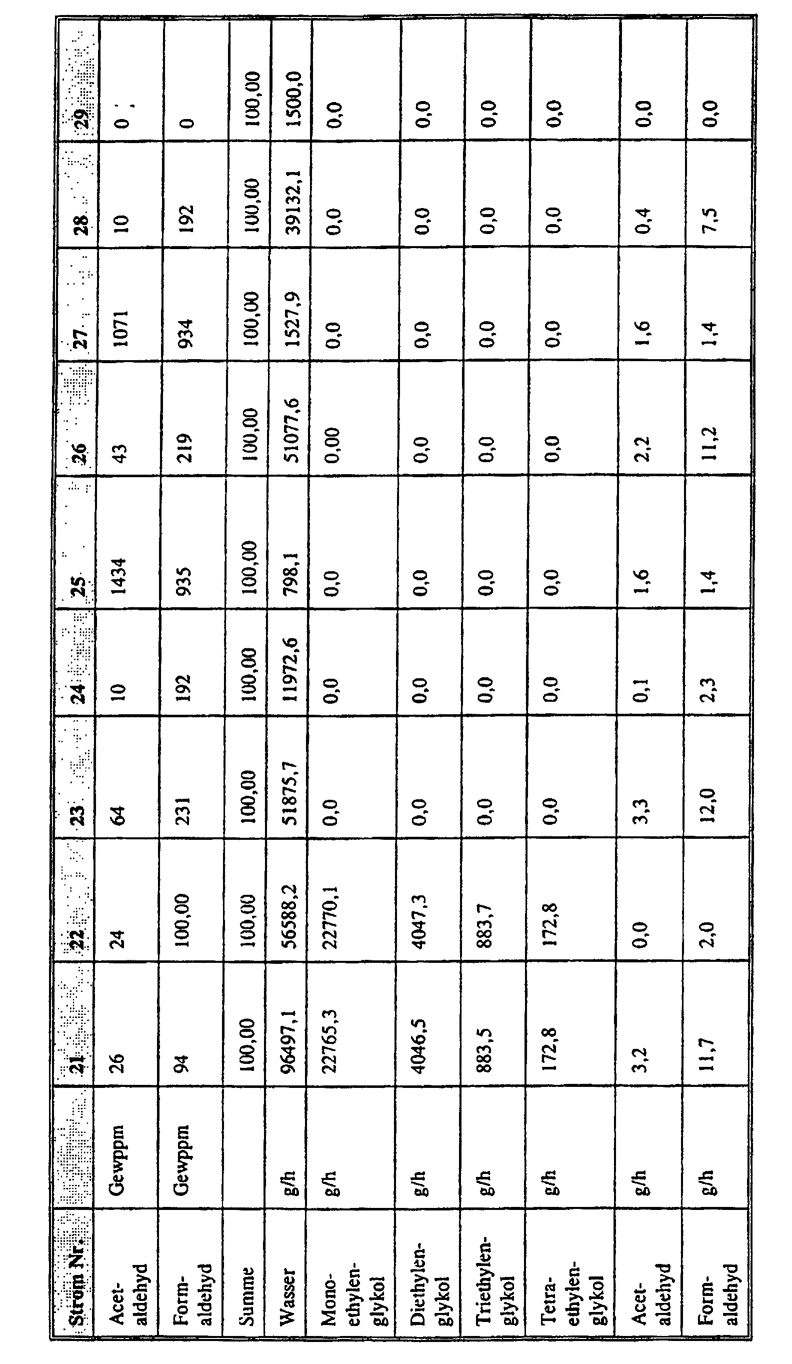

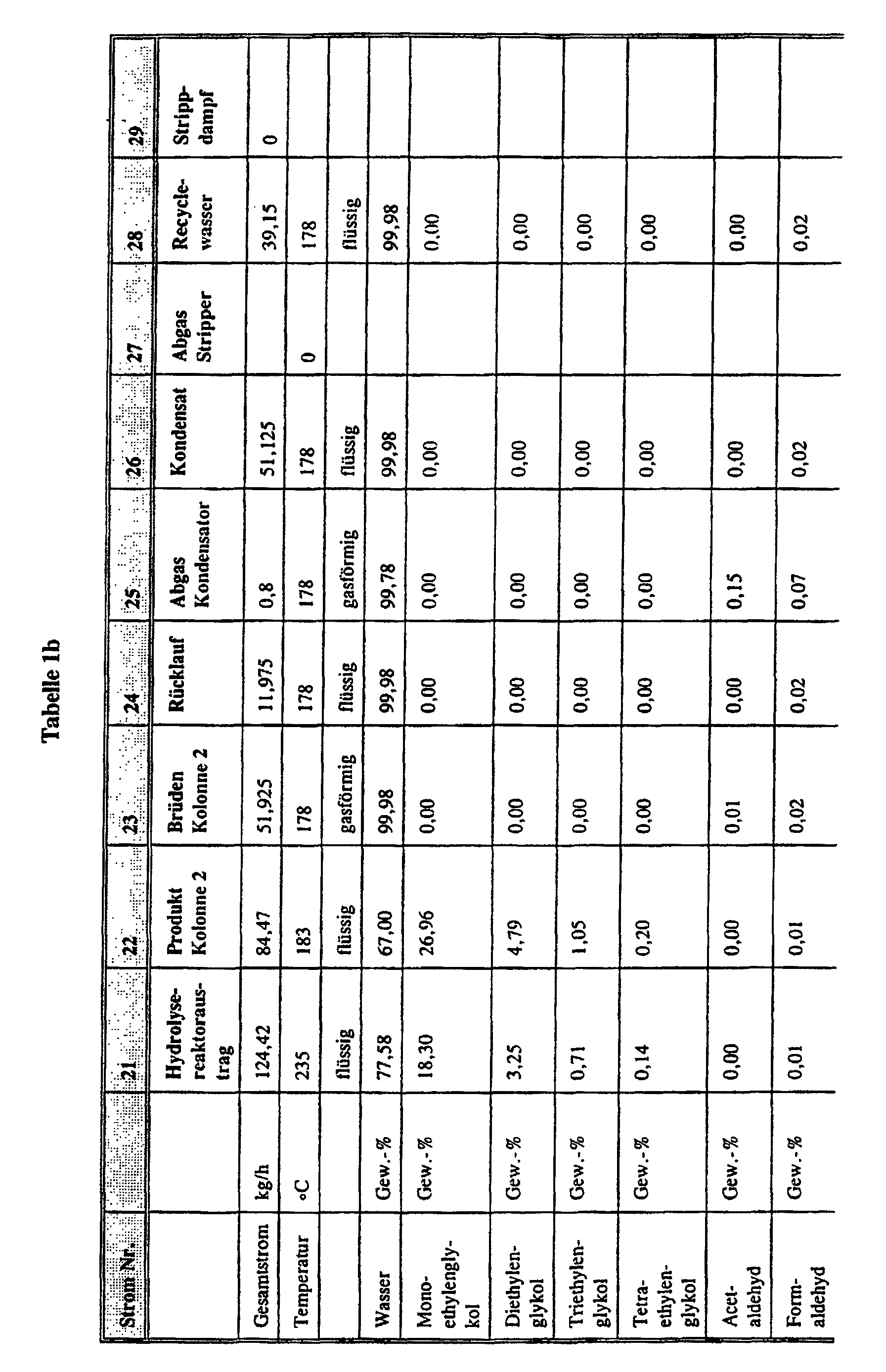

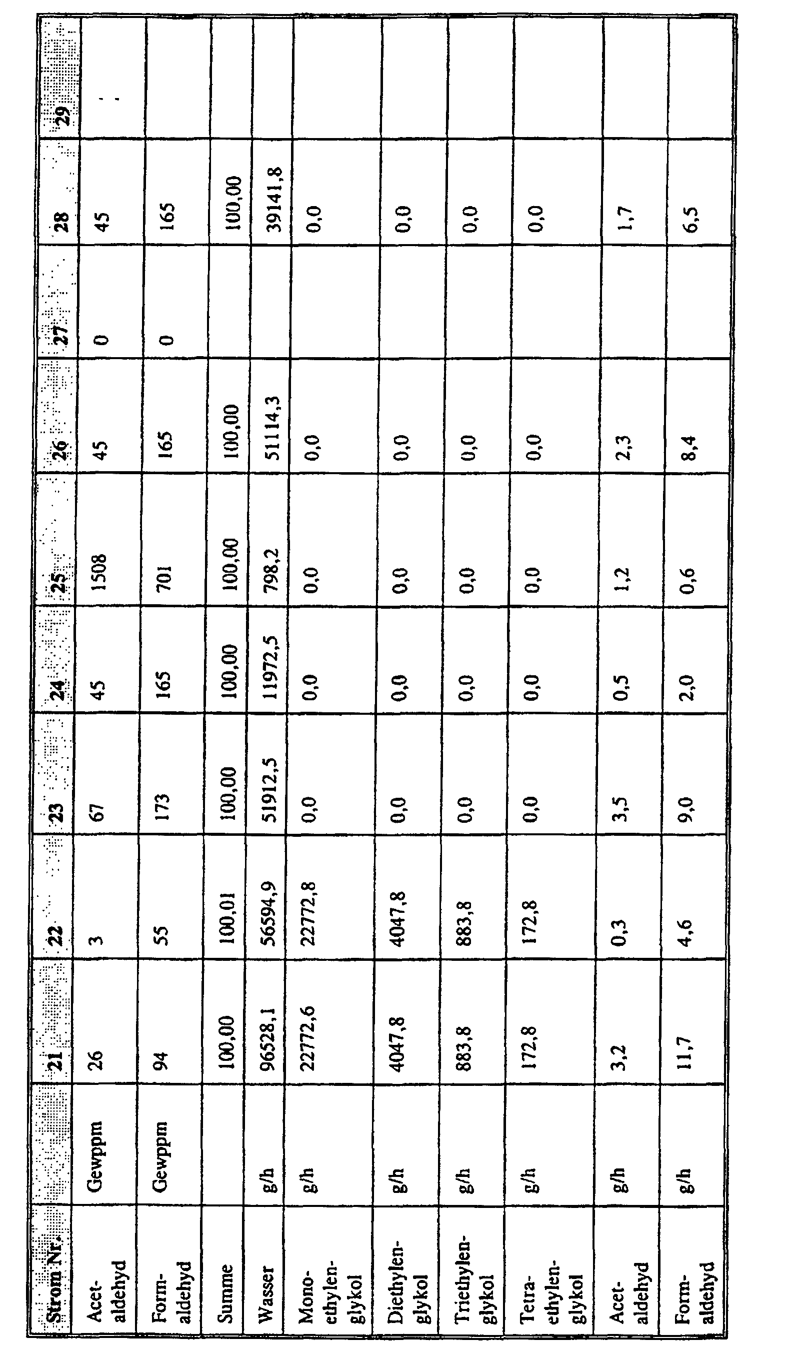

Nach dem erfindungsgemäßen Verfahren wird ein Produktstrom 22 aus der ersten Druckentwässerungskolonne 2 mit weniger Verunreinigungen (0,0 g/h Acetaldehyd und 2,0 g/h Formaldehyd) gegenüber dem Stand der Technik (0,3 g/h Acetaldehyd und 4,6 g/h Formaldehyd) erhalten.

An Nebenkomponenten werden erfindungsgemäß 1,1 g/h Acetaldehyd und 0,7 g/h Formaldehyd in Strom 25 sowie 1,6 g/h Acetaldehyd und 1,4 g/h Formaldehyd in Strom 27 ausgeschleust gegenüber nur 1,2 g/h Acetaldehyd und 0,6 g/h Formaldehyd in Strom 25 nach dem aus dem Stand der Technik bekannten Verfahren.

## Patentansprüche

1. Verfahren zur destillativen Gewinnung von hochreinem Monoethylenglykol aus dem Hydrolyseprodukt von Ethylenoxid durch Druckentwässerung, Vakuumentwässerung und anschließender Reindestillation, **dadurch gekennzeichnet, daß** die Druckentwässerungskolonnen oder mindestens die erste Druckentwässerungskolonne der Kaskade (2, 3, 4) einen Abtriebsteil mit mindestens einer Trennstufe aufweist wobei der Druck im Abtriebsteil mindestens 1 bar beträgt und wobei ein Teil des Kopfstroms der Druckentwässemngskolonne(n) (2, 3, 4) mit Abtriebsteil ausgeschleust wird.

2. Verfahren nach Anspruch 1, **dadurch gekennzeichnet, daß** der Druck im Abtriebsteil der Druckentwässerungskolonne (2) oder der ersten Druckentwässerungskolonne der Kaskade (2, 3, 4) 2 bis 30 bar beträgt.

3. Verfahren nach Anspruch 1 oder 2, **dadurch gekennzeichnet, daß** der Abtriebsteil 2 bis 10 Trennstufen aufweist.

4. Verfahren nach Anspruch 1 oder 2, **dadurch gekennzeichnet, daß** der Abtriebsteil 3 bis 6 Trennstufen aufweist.

5. Verfahren nach einem der Ansprüche 1 bis 4, **dadurch gekennzeichnet, daß** der Kopfstrom der Entwässerungskolonne(n) mit Abtriebsteil in einen Teilkondensator und/oder einen Stripper eingeleitet wird und daß der (die) an Nebenkomponenten angereicherte(n) gasförmige Strom (Ströme) ausgeschleust wird, (werden).

6. Verfahren nach Anspruch 5, **dadurch gekennzeichnet, daß** der Stripper als Wasserdampfstripper ausgebildet ist.

7. Verfahren nach einem der Ansprüche 1 bis 6, **dadurch gekennzeichnet, daß** Teilkondensator und Stripper bei Temperaturen über 90°C betrieben werden.

8. Verfahren nach Anspruch 7, **dadurch gekennzeichnet, daß** Teilkondensator und Stripper bei einer Temperatur zwischen 120 und 250°C betrieben werden.

## Claims

1. A process for the distillative recovery of high purity monoethylene glycol from the hydrolysis product of ethylene oxide by pressure dewatering, vacuum dewatering and subsequent purifying distillation, wherein the pressure dewatering columns or at least the first pressure dewatering column of the battery (2, 3, 4) has a stripping section with at least one separating stage, the pressure in the stripping section being not less than 1 bar, and a portion of the overhead stream of the pressure dewatering column(s) (2,3,4) having a stripping section being removed from the system.

2. A process as claimed in claim 1, wherein the pressure in the stripping section of the pressure dewatering column (2) or of the first pressure dewatering column of the battery (2,3,4) is within the range from 2 to 30 bar.

3. A process as claimed in claim 1 or 2, wherein the stripping section has from 2 to 10 separating stages.

4. A process as claimed in claim 1 or 2, wherein the stripping section has from 3 to 6 separating stages.

5. A process as claimed in any of claims 1 to 4, wherein the overhead stream of the dewatering column(s) having a stripping section is introduced into a partial condenser and/or a stripper and the gaseous stream(s) enriched with secondary components is (are) removed from the system.

6. A process as claimed in claim 5, wherein the stripper is a steam stripper.

7. A process as claimed in any of claims 1 to 6, wherein the partial condenser and the stripper are operated at above 90°C.

8. A process as claimed in claim 7, wherein the partial condenser and the stripper are operated at from 120°C to 250°C.

## Revendications

1. Procédé de production par distillation de monoéthylèneglycol de pureté élevée, à partir de l'hydrolysat de l'oxyde d'éthylène au moyen d'une déshydratation sous pression, d'une déshydratation sous vide, puis d'une distillation de purification, **caractérisé en ce que** les colonnes de déshydratation sous pression ou au moins la première colonne de déshydratation sous pression de la série (2, 3, 4) présente une zone de rectification dotée d'au moins un étage de séparation, la pression dans la zone de rectification s'élevant à au moins 1 bar, et une partie du courant de tête de la ou des colonnes de déshydratation sous pression (2, 3, 4) étant soutirée de la zone de rectification.

2. Procédé selon la revendication 1, **caractérisé en ce que** la pression dans la zone de rectification de la colonne de déshydratation sous pression (2) ou de la première colonne de déshydratation sous pression de la série (2, 3, 4) s'élève de 2 à 30 bars.

3. Procédé selon la revendication 1 ou 2, **caractérisé en ce que** la zone de rectification présente de 2 à 10 étages de séparation.

4. Procédé selon la revendication 1 ou 2, **caractérisé en ce que** la zone de rectification présente de 3 à 6 étages de séparation.

5. Procédé selon l'une quelconque des revendications 1 à 4, **caractérisé en ce que** le courant de tête de ou des colonnes de déshydratation dotées d'une zone de rectification est introduit dans un condenseur partiel et/ou un revaporisateur, et **en ce que** le ou les courants gazeux enrichis en composants secondaires sont éclusés vers l'extérieur.

6. Procédé selon la revendication 5, **caractérisé en ce que** le revaporisateur se présente sous la forme d'un revaporisateur à vapeur d'eau.

7. Procédé selon l'une quelconque des revendications 1 à 6, **caractérisé en ce que** le condenseur partiel et le revaporisateur sont mis en oeuvre à des températures supérieures à 90°C.

8. Procédé selon la revendication 7, **caractérisé en ce que** le condenseur partiel et le revaporisateur sont mis en oeuvre à une température comprise entre 120°C et 250°C.
